# EUROPEAN PATENT SPECIFICATION

(11) **EP 2 395 958 B1**
(45) Date of publication and mention of the grant of the patent: **13.12.2017**
(21) Application number: 10741696.8
(22) Date of filing: 11.02.2010
(51) Int. Cl.: A61F 13/02

(54) **DEVICES FOR MANIPULATING CIRCULATION IN THE CIRCULATORY SYSTEM OF A PATIENT**
VORRICHTUNGEN ZUR MANIPULATION DES KREISLAUFES IM KREISLAUFSYSTEM EINES PATIENTEN
DISPOSITIFS POUR LA MANIPULATION DE LA CIRCULATION DANS LE SYSTÈME CIRCULATOIRE D'UN PATIENT

(30) Priority: 17.04.2009 US 170107 P; 09.10.2009 US 250494 P; 06.03.2009 US 158341 P; 12.02.2009 US 151843 P; 03.12.2009 US 266327 P
(43) Date of publication of application: 21.12.2011
(73) Proprietor: Perfuzia Medical, Inc., Providence, RI 02906 (US)
(72) Inventor: BRINK-DANAN, Sagi, Providence RI 02906 (US); SCHUBERT, Shai, Y., Brookline MA 02446 (US)
(74) Representative: advotec.
(86) International application number: PCT/US2010/023832
(87) International publication number: WO 2010/093753

(56) References cited:
- WO-A1-02/38216
- WO-A1-2005/105175
- WO-A2-2006/114638
- US-A1- 2003 036 715
- US-A1- 2004 030 267
- US-A1- 2004 077 978
- US-A1- 2006 282 028
- US-A1- 2006 282 028
- US-A1- 2007 208 280

## Description

### BACKGROUND OF THE INVENTION

### 1. Field of the Invention

This invention relates to a device for manipulating circulation in the circulatory system of the body of a subject.

### 2. Description of the Related Art

Chronic lower extremity ulcers affect approximately 2.5 million to 4.5 million people in the United States. In addition to pressure ulcers, this growing clinical problem is most prominent among the elderly. Non-healing or slow healing wounds represent a major health burden and a drain on resources, and are sources of substantial disability, morbidity, and costs.

Multiple factors have been identified as contributors to impaired wound healing, such as ischemia, infection, advanced age, malnutrition, diabetes, and renal disease. Other conditions, such as cardiac and lung disease, decreased cognitive function, endocrine disease, gastrointestinal disease, hematologic disorders, incontinence, musculoskeletal problems, neurological disease, alcohol/drug abuse, immunosuppressives, chemotherapy, steroids, smoking, surgery as well as inadequate wound care have been implicated as well.

Wound healing involves a complex interaction between epidermal and dermal cells, the extracellular matrix, controlled angiogenesis, and plasma derived proteins, all coordinated by an array of cytokines and growth factors. This dynamic process is divided into three overlapping phases, inflammation, proliferation, and remodelling. Thrombus formation which requires interaction between endothelial cells, platelets, and coagulation factors achieves haemostasis after tissue injury. Trapped cells within the clot, predominantly platelets, trigger an inflammatory response by the release of vasodilators and chemoattractants and activation of the complement cascade.

Inflammation - In the early phase of inflammation, neutrophils predominate, removing bacteria and other foreign material from the wound by releasing enzymes and by phagocytosis. Later in the inflammatory phase, neutrophils reduce in number and are replaced by macrophages. Macrophages play a role in coordinating the transition from inflammation to proliferation through the release of soluble mediators, which include platelet-derived growth factor, tumor necrosis factor α, transforming growth factor β, and insulin growth factor 1.

Proliferation - Fibroblasts are the key cells involved in the production of the extracellular matrix. In addition to producing collagen, they produce tenascin, fibronectin, and proteoglycans such as hyaluronic acid resulting in the formation of granulation tissue. The combination of new tissue and contraction of surrounding tissues is essential for the healing of ulcers. While new matrix is synthesized, existing matrix in and around the wound margin is degraded by several enzyme systems such as matrix metalloproteinas-es and plasminogen activators. While some keratinocytes at the wound edge proliferate, others undergo a marked transformation to enable them to phagocytose debris and migrate across the wound bed. Keratinocyte migration coupled with wound contraction results in re- epithelialisation and wound closure.

Remodelling - Once closure of the wound has been achieved, remodelling of the resulting scar takes places over months or years, with a reduction of both cell content and blood flow in the scar tissue.

Restricted blood supply to a living tissue (ischemia) may result in failure of the tissue to function normally. Although ischemia may be the result of many different conditions, the underlining mechanism generally involves vascular dysfunction. Increasing blood flow (perfusion) to an ischemic tissue may facilitate the restoration of tissues functionality. One condition known to be effected by ischemia is chronic wounds. Insufficient circulation in the wound area results in impaired trafficking of effector cells and molecules to and from the wound area, leading to delayed healing or non-healing wounds. Increasing blood flow to the wound area may therefore enhance the healing of ischemic wounds.

Various devices have been proposed for placement on the skin for healing or drug delivery. U.S. Patent No. 3,853,121 describes a method for imparting vibration to the legs of a patient. U.S. Patent Application Publication No. 2009/0069728 discloses a therapeutic device for treatment of blood flow disorders. U.S. Patent Application Publication Nos. 2004/0077978 and 2009/0234258 and U.S. Patent No. 7,615,018 and PCT Patent Application Publication No. WO 02/065973 describe a treatment device that delivers mechanical vibrations to the limb of an animal or human. U.S. Patent Application Publication No. 2004/0167461 and U.S. Patent No. 7,643,874 disclose a dermal patch for transdermal or intradermal delivery of a substance. U.S. Patent Application Publication No. 2008/0234616 describes an inflatable compression dressing.

Document WO 2006/114638 A2 describes an apparatus for cleansing wounds in which irritant fluid from a reservoir connected to a conformable wound dressing and wound exudate from the dressing are moved by a pump for moving fluid through a flow path which passes through the dressing and a means for providing simultaneous aspiration and irrigation of the wound. The apparatus also comprises means to apply high frequency vibrational energy, e.g. ultrasound, to the wound bed. The ultrasound has frequencies between 20 kHz and 10 MHz.

Document WO 2005/105175 A1 discloses an apparatus for cleansing wounds including means to apply high frequency vibrational energy, e.g. ultrasound, to the wound bed. The ultrasound has frequencies between 20 kHz and 10 MHz. Still, there is a need for an apparatus for manipulating local and/or regional blood circulation such that the wound healing process can be enhanced.

### SUMMARY OF THE INVENTION

In one aspect, the present invention satisfies the foregoing needs by providing an apparatus for manipulating local and/or regional circulation, such as inducing vasodilation or vasoconstriction, by means of vibrational stimuli.

An increase in blood trafficking at the site of an ischemic wound can result in better mobilization of cells and molecules important for the wound healing process. Better trafficking of cells and biomolecules in the wound area may enhance the healing process resulting in improvement of non healing wounds and reduced healing time in slow healing wounds. The present invention provides a device, preferably designed as a stand alone unit, containing an adhesive flexible substrate containing a controller, a power source and one or more vibration actuators that can comprise one unit to be applied at the wound area. The device results in increased blood circulation at the wound area. The unit is designed to be flexible so it will fit around or near the wound area and will not require additional involvement such as an external power supply. By applying the unit around or next to the wound, the device will stimulate blood flow by applying vibration stimuli to the skin surrounding the wound. The vibrations from the device stimulate the tissue and can result in vasodilatation and in increased blood flow.

In another aspect not covered by the claims, the foregoing needs are satisfied by providing an apparatus for the local enhancement of blood circulation. The device includes a dermal patch having a porous layer and a fluid (e.g., air) sealed outer layer with a vacuum tube connector. Hence, negative pressure can be customized for application to different body locations by attaching the porous layer to a body location and connecting a source of negative pressure to the vacuum tube connector.

These and other features, aspects, and advantages of the present invention will become better understood upon consideration of the following detailed description, drawings and appended claims.

### BRIEF DESCRIPTION OF THE DRAWINGS

Figure 1 is a block diagram of an example embodiment of a device according to the invention for manipulating circulation in the circulatory system of the body of a subject.
Figure 2 is a perspective view of an embodiment of a device according to the invention for manipulating circulation in the circulatory system of the body of a subject.
Figure 3 is a rear view of a left human leg showing a wound in the calf.
Figure 4 is a top view of a wound map used in preparing another embodiment of a device according to the invention for manipulating circulation in the circulatory system of the body of a subject.
Figure 5 is a top view of an embodiment of a device not covered by the claims prepared using the wound map of Figure 4.
Figure 6 is a rear view of the leg of Figure 3 showing the device of Figure 5 positioned adjacent the wound in the calf.
Figure 6A is a cross-sectional view taken along line 6A-6A of Figure 6.
Figure 7 is a perspective view of another embodiment of a device not covered by the claims with a portion of the perimeter sealing tape not being shown.
Figure 8 is a perspective view of yet another embodiment of a device not covered by the claims with a portion of the perimeter sealing tape not being shown.
Figure 9 is a bottom perspective view of still another embodiment of a device not covered by the claims.
Figure 10 is a bottom perspective view of yet another embodiment of a device not covered by the claims.
Figure 11 is a left, rear perspective view of a human right foot and ankle having still another embodiment of a device according to the invention positioned in the ankle region and the arch region.
Figure 12 is a bottom view of a human right foot having yet another embodiment of a device according to the invention positioned in the arch region.
Figure 13 is a graph showing THI (total hemoglobin index) versus time for a patient using a device according to the invention positioned on the sacrum.
Figure 14 is a graph showing tissue oxygenation versus time for a patient using a device according to the invention positioned on the sacrum.
Figure 15 shows graphs of tissue oxygenation (StO2) versus time and total hemoglobin (THI) versus time for a patient using a device according to the invention positioned on the sacrum.

Like reference numerals will be used to refer to like parts from Figure to Figure in the following description of the drawings.

### DETAILED DESCRIPTION OF THE INVENTION

In one example embodiment, a method is provided not covered by the claims and an apparatus for manipulating local and/or regional circulation in a subject comprising delivery of at least one signal of vibrational stimulus to at least one area of the body of the subject. By delivering the vibrational stimuli to an area that is adjacent to a wound in accordance with this method, the circulation to certain body parts, preferably surrounding the wound, is changed. Vasodilation or vasoconstriction is induced in target organs (although the target organ is not necessarily the site of stimulation) or the wound area, resulting in increased or decreased blood perfusion in the target tissue or wound area. Clinical benefits effectuated using the method not covered by the claims include, but are not limited to: improved perfusion for limb ischemia, improved perfusion for ischemic wounds and pressure wounds (decubitus ulcers), improved perfusion for peripheral arterial disease (PAD) and peripheral vascular disease (PVD) patients and restricted blood flow to inflamed or injured body parts, improved perfusion to diabetic foot ulcers, improved perfusion to ischemic tissues resulting from surgery, including but not limited to plastic surgery and flap surgery, and improved perfusion to injured or inflamed muscle tissue.

In addition to chronic wounds, peripheral ischemia may lead to the development of other conditions including neuropathy characterized by progressive loss of nerve fibers. Enhancement of local perfusion by delivering the vibrational stimuli to an area in or adjacent to a neuropathic tissue with this method may be used for the treatment of neuropathy by increasing perfusion to ischemic nerves resulting in reduced nerve fiber loss and improved nerve function.

The vibrational stimulus signal can be comprised of a single or multiple waveforms, and it can change over time. The preferred amplitude of the vibrational stimulus signal is from about 1 µm to about 15 millimeters, and more preferred is an amplitude of the vibrations ranging between 0.001 millimeters and 2.5 millimeters. The preferred waveform is sinusoidal. The frequency of the vibrational stimulus signal is preferably in the range of about 1 Hz to about 15,000 Hz. The force of the vibrational stimulus is preferably in the range of about 0.001 Newtons to 100 Newtons. These ranges of vibrational stimuli can have vasoconstrictive and/or vasodilative effect.

In another example embodiment of the invention, the vibrational stimulus signal is delivered by a wearable system. The system can be worn directly on the body of the subject, or over one or more layers of clothing. The term "wearable system" as used herein refers to any structure capable of holding in place some or all of the components described hereafter, in a desired location on the subject's body.

In yet another example embodiment of the invention, the vibrational stimulus signal is delivered by attaching the device directly to the patient's body at the wound location by gluing the device with adhesive or adhesive patches such as polyurethane and fabric based patches. In other embodiments, the device is held pressed against the skin by other mechanisms, for example: one or more elastic bands, Velcro™ hook and loop fasteners, tape, clips, or bandaging.

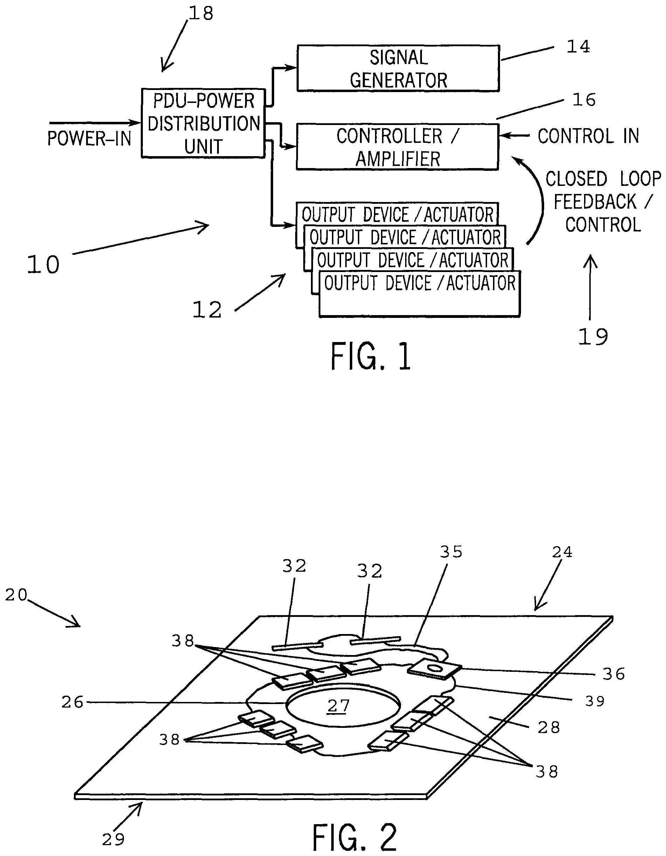

Different embodiments can be designed as to be capable of adhering some or all components of the system in place on the upper body of an individual including torso, shoulder, arm, elbow, neck, wrist and hand, as well as on the lower body including waist, hip, leg, knee, ankle and foot. As shown in the block diagram of Figure 1 , the wearable system 10 can include one or more output devices 12 which deliver the vibrational stimuli, a signal generator 14, a controller 16 and a power unit 18. The signal generator 14, controller 16, power unit 18 and other electric / electronics components such as an amplifier may be implemented as separate units or incorporated into a single component. The output devices 12 can be repositionable and/or detachable to the wearable unit. The signal generator 14, the controller/driver 16, and the power unit 18 can be detachable from a substrate. The system 10 can be controlled locally or remotely using a wired or wireless remote control unit. Various parameters of the system will be modifiable by means of the local or remote control unit, such as the frequency, amplitude, force, and duration of the stimuli. The system 10 can also include means for calibration of the vibrational stimulus signal in an initial step of utilization, or continuously throughout the operation, in real-time or near-real-time. It can also include one or more closed-loop feedback / control circuits 19 to monitor the effect on blood flow or other physiological parameters and modify the output vibrational stimuli delivered to the subject in real-time or in near-real-time. Measurements of stimuli on physiological parameters can be achieved by one or more vibration or perfusion sensors such as piezo-film sensors, temperature sensors, photoplethysmographic sensors, strain-gauge plethysmography sensors, laser Doppler sensors, ultrasound sensors, or other such sensors. The wearable system 10 can be powered from an electrical wall socket, a portable DC power source such as batteries, other power sources, or a combination of several power sources.

In another example embodiment of the invention, all of the components 12, 14, 16, 18 and 19 are embedded within one system employed topically, locally or regionally, adjacent to or surrounding the wound area. In alternative embodiments one or more of the components, for example, the power supply 18 or the controller 16, can be external to the wearable substrate. For example, in a bed-side console and electrically connected via wire(s) or wirelessly to the wearable substrate.

The components of the device can include an adhesive substrate comprising a flexible material such as silicone, polyurethane, nylon, fabric, paper, or other polymer material for use as the substrate. With at least one of its sides carrying adhesive, the substrate can attach to the skin or to other bandaging materials or tight clothing and remain attached unless removed. The device components are located on or within the substrate and include the vibration actuators, controller and power sources.

In one example embodiment, the output devices can be of an electromechanical nature such as linear electro-magnetic actuators, magnetostrictive actuators (DMA), hydro-pressure, asymmetric mass motors, voice coils, electro- active polymers (EPAM) or piezo-electric actuators, or other such devices.

In another embodiment, the output devices can be of pneumatic or hydraulic nature. These can include gas-containing resonating elements or fluid- containing resonating elements that will create and sustain the mechanical vibrational stimuli; or they can include gas-containing pouches or fluid-containing pouches or chambers that will deliver the vibrational stimuli to the subject's body surface in combination with electromechanical elements that will create the vibrational stimuli and deliver them to the pouches.

In yet another embodiment, a method not covered by the claims and an apparatus are provided for manipulating local and/or regional circulation in a subject comprising delivery of thermal stimuli such as heat or cold combined with delivery of at least one signal of vibrational stimulus to at least one area of the subject's body. In one embodiment, the application of heat or cold is performed by means of electrical and electronic components such as heating coils and thermoelectric coolers. In other embodiments, thermal stimuli can be achieved by other means, such as mechanical, chemical or other methods of heating and cooling surfaces.

A device according to the invention can be designed for single use, or for repeated use, for example by employing rechargeable or external power sources, or by using the same electronic apparatus while replacing the skin attachable element (adhesive substrate).

In one example method of using the invention, the system is designed to be used while the subject is in rest (e.g., seating or laying), or in motion, performing mild physical activities such as walking. The device is placed in proximity to the location where increased circulation is desired with the vibration actuator or actuators in direct contact with the skin, in close proximity to the skin or over a reasonably thin layer of bandaging or tight clothing. The device will operate for defined periods of time defined by the specific needs of the subject. For example, the device will be used for fifteen minutes with inactive intervals of fifteen minutes.

The apparatus used for performing the method of the present invention provides multiple improvements over known units used for manipulating local circulation by means of vibratory stimuli. For example, the present invention provides the ability of the specific design and specification to serve as a therapeutic instrument for targeted indications such as peripheral artery disease and chronic wounds. Current units are not wearable, most commonly they are hand-held and applied by hand to a specific area of the body; they are designed for use on a particular body area - and on it alone - such as the calf or the foot; they employ large vibrating surfaces resulting in large-area or even whole-body vibration; alternatively they employ very small applicators for highly localized stimuli; they typically require AC power supply limiting their use and portability, and they are not designed for continuous treatment - only for short sessions once or more a day; finally, known units do not include feedback loops (physiological or mechanical), and do not combine other types of stimulus together with the vibratory stimulus.

The current invention provides a system that is fully wearable and portable, is battery-operated, and can be easily applied to any part of the body. Furthermore, the current invention has a local/regional effect and provides continuous therapy over any desired period of time (e.g., from minutes to hours to weeks to months), not limited to short repetitive sessions.

For all of the above reasons, the current invention provides significant improvements over existing devices, and is particularly suitable for the therapeutic purposes described herein.

From an application point of view, the study of vibrations effect on circulation is in most cases focused on the damaging effect of vibrations induced by industrial machinery, or on the effect of vibrations on growth of bone mass. Current studies focus on short term application of vibrations (seconds to minutes). The current invention and the research that has lead to it are geared towards continuous application of vibrations and their specific therapeutic purposes as described herein.

Figure 2 shows one non-limiting example configuration for a device 20 according to the invention for local enhancement of circulation and alleviation of pain in lower limbs, caused due to peripheral artery disease, or an ischemic wound in the leg. The device 20 includes a substrate 22 having a perimeter 24 and an opening 26 which surrounds a wound location 27. The device 20 includes an upper surface 28 and lower surface 29. The lower surface 29 of the device 20 can be coated with an adhesive layer for attachment to a section of the body of a subject. The wound at the wound location 27 can be, for example, a diabetic ulcer, ischemic ulcer, venous ulcer, arterial ulcer, ischemic wound, pressure wound, injury, surgery, burn, inflammation, muscle injury, or internal injury.

The substrate 22 can be formed, for example, from a material selected from silicone, synthetic foam, polyethylene, polyurethane, polyvinyl chloride, plastic, nylon, thermoplastic polyurethane, polypropylene, fabric, hydrogel, collagen, alginate, gelatin, or combinations thereof. The adhesive layer can comprise, for example, a compound selected from urethanes, epoxies, urea, melamine, polyamides, polyesters, polyethers, polyolefins, polyvinyls, sulfonates, acrylates, methacrylates, and combinations thereof.

The substrate 22 can be of an area size and shape designed based on the location of ischemic ulcers. For example, a foot ulcer will require a different design compared with facial or back wounds. While the opening 26 of the embodiment of Figure 2 completely surrounds the wound location 27, other shapes for the substrate 22 and opening 26 such that the substrate 22 partially surrounds the wound location 27 are possible. For example, the substrate 22 may have a crescent shape, or the substrate 22 can be split into two pieces such as at a horizontal line of symmetry.

The device 20 includes an electrical power source such as batteries 32, which can be non-rechargeable or rechargeable. The batteries 32 are connected via electrical lines 34,35 to a controller 36, which can be a programmable microprocessor. The device 20 includes wide range frequency vibration energy generators 38 which are in electrical communication with the controller 36 via electrical line 39. The vibration generators 38 can transmit vasodilating or vasoconstricting frequencies to the tissue (e.g., foot sole or lower leg) surrounding the ischemic wound resulting in enhancement of local circulation. The vibration generators 38 can be an actuator such as a piezo electric actuator. The controller 36 executes an internally or externally stored program for providing electrical signals to the vibration generators 38 for adjusting vibrational stimulus frequency, amplitude, force, and/or timing of the vibration energy generators 38.

The vibrational stimulus signal in the device 20 of Figure 2 can be a single or multiple waveforms, sinusoidal, square, pulse, triangle or combination thereof, can be unidirectional or multidirectional, and can change over time. The preferred amplitude of the vibrational stimulus signal is from about 1 µm to about 15 millimeters. The frequency of the vibrational stimulus signal is preferably in the range of about 1 Hz to about 15,000 Hz. The force of the vibrational stimulus is preferably in the range of about 0.001 Newtons to 100 Newtons.

Optionally, a vibration sensor or a perfusion sensor can be applied to tissue adjacent the substrate 22. The vibration or perfusion sensor monitors the physiological response to specific frequency and power of stimulus. The vibration or perfusion sensor is in electrical communication with the controller 36, which can execute a stored program for adjusting vibrational stimulus frequency and power based on electrical signals representing readings taken by the vibration or perfusion sensor. The controller 36 can integrate data collected by the vibration or perfusion sensor to adjust the vibration frequency and energy transmitted by the vibration generators 38. The vibration feedback can be a skin sensor, or a sensor that is embedded within the vibration inducing element, or a sensor that is attached to the vibration inducing element, or software and/or hardware within the controller 36 that detects current and/or voltage draw by the vibration inducing elements and can infer on the element's performance based on that.

The controller 36 can be programmed with various algorithms to control the vibration generators 38. In one non-limiting example algorithm, the controller 36 executes an internally or externally stored program to provide a first electrical signal for a first time duration to the vibration inducing element. The first electrical signal controls the frequency and/or amplitude and/or force of vibrations of the vibration inducing element. After the first time duration ends, the controller 36 either ceases providing the first electrical signal to the vibration inducing element or decreases or increases the intensity of the first electrical signal provided to the vibration inducing element for a second time duration. After the second time duration ends, the controller 36 resumes providing the first electrical signal to the vibration inducing element or increases or decreases the intensity of the first electrical signal provided to the vibration inducing element for a third time duration. Optionally, one or more of the frequency and the amplitude and force of vibrations of the vibration inducing element during the third time duration can be different than the frequency and the amplitude of vibrations of the vibration inducing element during the first time duration.

In another non-limiting example algorithm, the controller 36 ceases providing the first electrical signal to the vibration inducing element or decreases or increases the intensity of the first electrical signal provided to the vibration inducing element for a fourth time duration, and thereafter resumes providing the first electrical signal to the vibration inducing element or increases or decreases intensity of the first electrical signal provided to the vibration inducing element for a fifth time duration.

In still another non-limiting example algorithm, the controller 36 provides a first electrical signal to the vibration inducing element wherein the first electrical signal controls the frequency and amplitude and force of vibrations of the vibration inducing element, and varies the first electrical signal to the vibration inducing element such that at least one of the frequency or amplitude or force of vibrations of the vibration inducing element is varied.

In yet another non-limiting example algorithm, the controller 36 provides a first electrical signal for a first time duration to the vibration inducing element for controlling the frequency and/or amplitude and/or force of vibrations of the vibration inducing element, and can vary the first electrical signal provided to the vibration inducing element based on a second electrical feedback signals received from the perfusion sensor or the vibration sensor applied to tissue adjacent the substrate 22.

In still another non-limiting example algorithm, the controller 36 provides a first electrical signal for a first time duration to a first vibration inducing element for controlling the frequency and/or amplitude and/or force of vibrations of the first vibration inducing element, ceases providing the first electrical signal to the first vibration inducing element for a second time duration, and thereafter provides a second electrical signal to the second vibration inducing element for a third time duration for controlling the frequency and amplitude and force of vibrations of the second vibration inducing element.

It can be appreciated that the programmable controller 36 allows for an infinite number of programs that provide for various time periods of operation or non-operation at various frequencies and amplitudes and forces for the vibration generators 38, either individually or as a group of vibration generators. For example, during a first time duration the vibration generators 38 may operate for one minute to two hours in which the vibration generators 38 transmit vibrations to the skin at a first frequency and amplitude and force, and then during a second time duration of one minute to six hours the vibration generators 38 do not transmit vibrations to the skin. The timing of operation (e.g., a sequence of time durations) can be repeated, for example, over a number of days, weeks, or months.

A kit not covered by the claims can include one or more of the following: the batteries 32, the electrical lines 34, 35, the controller 36, vibration generators 38, the electrical line 39, the substrate 22, a perfusion sensor, a vibration sensor, and instructions for use. The batteries 32, electrical lines 34,35, controller 36, vibration generators 38, and electrical line 39 can be attached to the upper surface 28 of the substrate 22 with suitable attachment means such as an adhesive, fasteners such as Velcro™ hook and loop fasteners, or a designated pouch. Alternatively, the batteries 32, electrical lines 34, 35, controller 36, vibration generators 38, and electrical line 39 can be embedded between the upper surface 28 of the substrate 22 and a second substrate (not shown). Alternatively, the batteries 32, electrical lines 34, 35, controller 36, vibration generators 38, and electrical line 39 can be encased in a hard or soft shell, which is in turn attached to the upper surface 28 of the substrate 22 or embedded between the upper surface 28 of the substrate 22 and a second outer substrate. By removably attaching (such as with Velcro™ hook and loop fasteners) the batteries 32, electrical lines 34,35, controller 36, vibration generators 38, and electrical line 39 to the upper surface 28 of the substrate 22, it is possible to reuse the electrical components on disposable substrates that can be changed at various intervals (e.g., every day). Alternatively, the batteries 32 and controller 36 can be placed in a suitable housing and an electrical line can be plugged into a suitable plug on the substrate 22 for electrical connection with the vibration generators 38. The housing can be attached to a bed support, a belt or a body part (e.g., arm, leg), or placed in a container such as a pocket or a hand bag.

The device 20 can be used at the same time with other treatments. For example, the device 20 can be used at the same time with hydrogel matrices (which can be separately) applied over the wound wherein the hydrogel comprises one or more of polylactic acid, polyglycolic acid, other polyhydroxy acids, copolymers of two or more polyhydroxy acids, polyorthoesters, polyanhydrides, gelatin, collagen, cellulose, derivatized cellulose, chitosan, alginate, thiol modified hyaluronan, and combinations or copolymers thereof. The hydrogel can also be made of synthetic material such as silicone plastic or fabric. The other treatment can include growth factors like vascular endothelial growth factor of platelet derived growth factor, fibroblast growth factor, cell therapies like stem cells, progenitor cells, fibroblasts or any other cell, gene therapies, and combinations thereof. The hydrogel matrix can include a bioactive agent selected from growth factors, stem cells, progenitor cells, fibroblasts, gene therapies, and combinations thereof. The substrate 22 can include a bioactive agent selected from cells, precursors, drugs, enzymes, organic catalysts, ribozymes, organometallics, proteins, glycoproteins, peptides, polyamino acids, antibodies, nucleic acids, steroidal molecules, antibiotics, antimycotics, cytokines, growth factors, carbohydrates, oleophobics, lipids, pharmaceuticals, therapeutics, and mixtures thereof. The substrate 22 can include a cosmetic for cosmetic uses in the enhancement of skin appearance. The other treatment can be selected from negative pressure, hyperbaric oxygen, compression devices, shock wave and ultrasound devices, and electric current stimulation.

The device 20 can be used in combination with other devices such as devices for applying negative pressure below the support, hyperbaric oxygen devices, compression devices, shock wave devices, heating devices, cooling devices, light emitting devices, ultrasound devices, and electric current stimulation devices. The device can be used in combination with wound negative pressure therapy, a skin system, a skin implant, a biological or bioactive wound dressing, a drug delivery wound dressing, a wound drainage system, and combinations thereof.

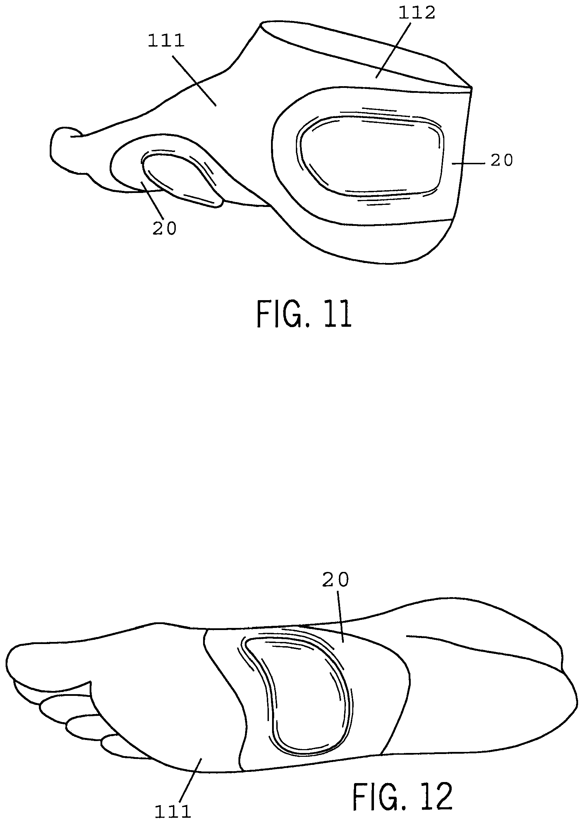

Figure 11 is a left, rear perspective view of a human right foot 111 and ankle 112 having a device 20 according to the invention positioned in the ankle region and the arch region. Figure 12 is a bottom view of a human right foot 111 having a device 20 according to the invention positioned in a foot sole plantar configuration. The device 20 can induce vasoconstriction or vasodilatation in the foot and/or ankle by means of vibrational stimuli. The device is shaped to fit right foot 111 and/or ankle 112.

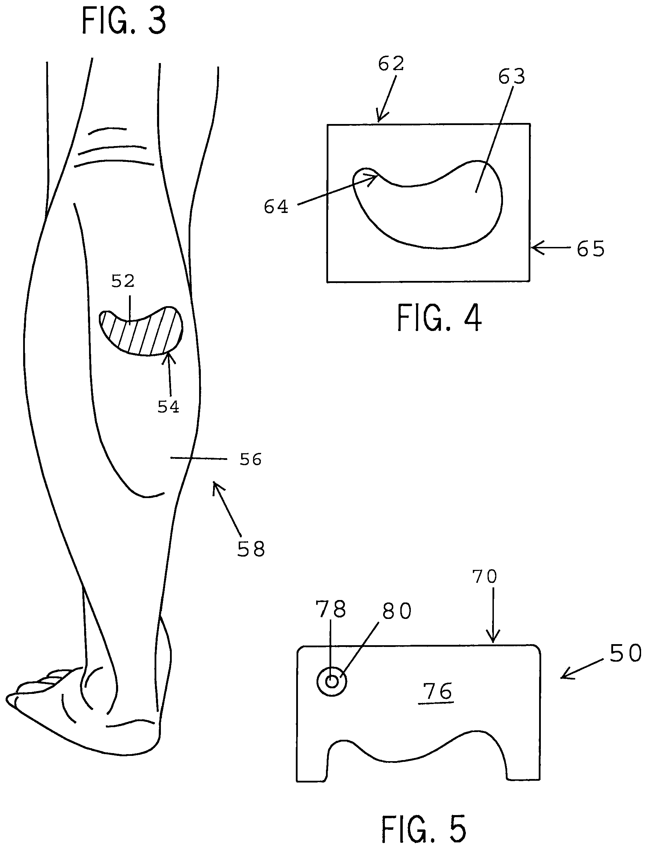

Turning now to Figures 3-10, there is shown another embodiment of a device 50 not covered by the claims. The device 50 can be called a Shape and Surface Adjustable Negative Pressure Applicator (SSANPA). The SSANPA device 50 provides a method for the application of negative pressure to the skin surface where the shape of the device 50 can be customized to the desired anatomy and area to be exposed to negative pressure. The device 50 is especially appropriate for applying negative pressure (vacuum) to normal skin and/or skin of the peri-wound area.

Referring now to Figures 3 and 4, a chronic wound 52 having a perimeter 54 is diagnosed on the calf 56 of the lower leg 58 of a human patient. Following diagnosis of the chronic wound 52, the wound measurements (e.g., the perimeter 54 of the wound 52) can be taken from the patient by direct measurement or by imaging. These measurements can be used to create wound map 62 as shown in Figure 4. The wound map 62 has an opening 63 with an inner edge 64 that corresponds to the perimeter 54 of the wound 52. The wound map 62 has an outer perimeter 65. A flexible plastic material is suitable for forming the wound map 62.

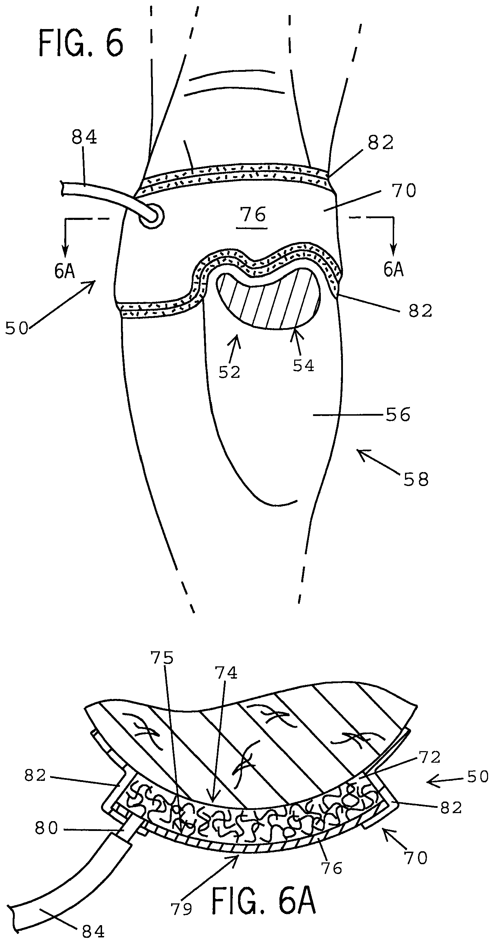

Turning now to Figures 5, 6 and 6A, a non-limiting example device 50 created using the wound map 62 is shown. The device 50 includes a flexible dermal patch 70 dimensioned for covering a section of the body of the subject. The patch 70 including a porous layer 72 structured such that fluid (e.g., air) can pass from a first side 74 of the porous layer 72 to an opposite second side 75 of the porous layer 72. The patch 70 further includes a fluid impermeable outer layer 76 covering the second side 75 of the porous layer 72. The patch 70 further includes a fluid passageway 78 extending from the second side 75 of the porous layer 72 to an outer surface 79 of the outer layer 76. The fluid passageway 78 terminates at a hollow connector 80 at the outer surface 79 of the outer layer 76. While the embodiment shown includes one fluid passageway that terminates at a hollow connector, more than one fluid passageway that terminates at a hollow connector can be provided in the device 50.

The dermal patch 70 can be supplied in an uncut rectangular shape similar to the outer perimeter 65 of the wound map 62. The outer perimeter 65 of the wound map 62 is aligned with the outer perimeter of the uncut dermal patch and the dermal patch is cut to follow the inner edge 64 of the wound map 62 (see Figure 4). The dermal patch 70 can be cut approximately in half to form the example dermal patch 70 shown in Figures 5 and 6. The shaped flexible dermal patch 70 is placed next to the wound 52 and a sealing tape 82 is applied along the dermal patch outer perimeter edges covering both the flexible dermal patch 70 and the skin of the calf 56 of the lower leg 58 of a human patient. A vacuum pump can be connected to the connector 80 by way of tubing 84.

In one form, the sealing tape 82 is a flexible, stretchable tape having a width ranging from 0.1 centimeters to 30 centimeters and more preferably 0.5 centimeters to 5 centimeters. The thickness of the tape 82 can be 0.001 millimeters to 5 millimeters, and more preferably 0.01 millimeters to 2.5 millimeters wherein the tape is coated with adhesive on one side such as when attached to both the perimeter of the dermal patch 70 and the skin, it will result in air sealing of the dermal patch 70. The sealing tape adhesive composition can include urethane, epoxy, urea, melamine, polyamide, polyester, polyether, saturated or unsaturated polyolefin, polyvinyl, sulfonate, acrylate or methacrylate compounds and/or combinations thereof. The substrate of the sealing tape 82 can include a polymer material, latex, rubber, silicone, fabric, cellulose, or combinations thereof.

In another embodiment, the sealing tape can be replaced with an adhesive film fully covering the flexible dermal patch 70 and overlapping the skin to form an air tight chamber. The adhesive film may contain a gas and liquid connector (such as connector 80) to allow connection to a vacuum pump. The thickness of the adhesive film can be 0.001 millimeters to 5 millimeters, and more preferably 0.01 millimeters to 2.5 millimeters wherein the tape is coated with adhesive on one side such as when attached to both the perimeter of the dermal patch 70 and the skin, it will result in air sealing of the dermal patch 70.

The adhesive film adhesive composition can include urethane, epoxy, urea, melamine, polyamide, polyester, polyether, saturated or unsaturated polyolefin, polyvinyl, sulfonate, acrylate or methacrylate compounds and/or combinations thereof. The substrate of the sealing tape 82 can include a polymer material, polyurethane, nylon, latex, rubber, silicone, fabric, cellulose, or combinations thereof.

For example, the products OpSite™ made by Smith and Nephew and Tegaderm™, a nylon film made by 3M, can be used for sealing film. The product OpSite™ is a semi-permeable, adhesive-coated polyurethane film. Sealing film is approximately 0.003 inches (0.076 mm) thick, however, it is within the scope of this disclosure to include any occlusive or semi-occlusive film having another thickness. The sealing film is provided to create a sealed environment below the film and around the dermal patch 70 in which a vacuum or negative pressure can be maintained.

In the embodiment shown in Figures 6 and 6A, the device 50 serves as an apparatus within which vacuum is created and applied to the skin of the calf 56 of the lower leg 58 of a human patient above the wound 52. It can be appreciated that the device 50 can be used to apply vacuum to other areas of skin on the body of the patient. Since the skin or tissue in contact with the device 50 serves as a sealing wall at the bottom of the device 50, negative pressure inside the device 50 is applied to the skin or tissue serving as a sealing wall to the device 50. When the tubing 84 is connected to the connector 80 and a source of negative pressure (e.g., a vacuum pump), negative pressure can be inflicted on the body area under the dermal patch 70 such that blood flow is increased in the body area. Negative pressure is applied to the dermal patch 70 or dermal patch 70a through the connector 80 using a vacuum pump to induce negative pressure ranging from 1 to 2000 mmHg, and more preferably between 25 and 500 mmHg.

The combination of the porous layer 72 and outer layer 76 of the device 50 is thin (preferably between 1 and 10 millimeters, but can also be between 10 millimeters and 500 millimeters or more). The porous layer 72 can be made of, for example, an elastic polymeric material such as open-cell polyurethane foam and open-cell polyvinyl alcohol foam, polyethylene, Ether-Like-Ester, polystyrene or other synthetic, biological or biodegradable polymers, fabric, layers of different polymers, layers of fabric and/or combinations thereof, preventing the dermal patch 70 from collapsing under normal negative pressure used during negative pressure treatment, yet allowing flexibility for bending and cutting such that the dermal patch 70 can be adjusted to cover and bind to different shapes and topographies. The porous layer 72 of the dermal patch 70 allows fluid (e.g., air) flow. In one form, the thickness of the porous layer 72 is between 0.1 centimeters and 10 centimeters, and more preferably between 0.2 millimeters and 2 centimeters.

In one embodiment, the base surface (skin contacting side) of the porous layer 72 is at least partially coated by an adhesive layer allowing the attachment of the dermal patch 70 to the skin or tissue. In another embodiment, the base surface of the porous layer 72 is not coated with adhesive. The top side of the outer layer 76 of the dermal patch 70 can be sealed to fluids (e.g., air) by applying a thin layer of air sealing polymer or other material that will prevent fluid from crossing the top barrier. The outer layer 76 can be made of synthetic or natural polymer such as rubber, latex, silicone, nylon or other flexible polymeric material, fabric and/or combination thereof.

The flexible sealing tape 82 is used to seal the dermal patch 70 following attachment of the porous layer 72 to the body. The sealing tape 82 can be made of thin elastic polymer such as latex (vinyl acetate, styrene-butadiene, acrylates) coated with adhesive on one side. The sealing tape 82 is designed with maximal elasticity to allow it to conform with the shape and topography of the dermal patch 70 following its attachment to the skin or tissue. The sealing tape 82 is preferably in a width which will allow convenient and safe attachment both to the dermal patch 70 as well as to the skin or tissue. In one example embodiment, the sealing tape 82 may be of a size that will entirely cover the vacuum dressing thereby it can serve as a top sealant for the vacuum dressing.

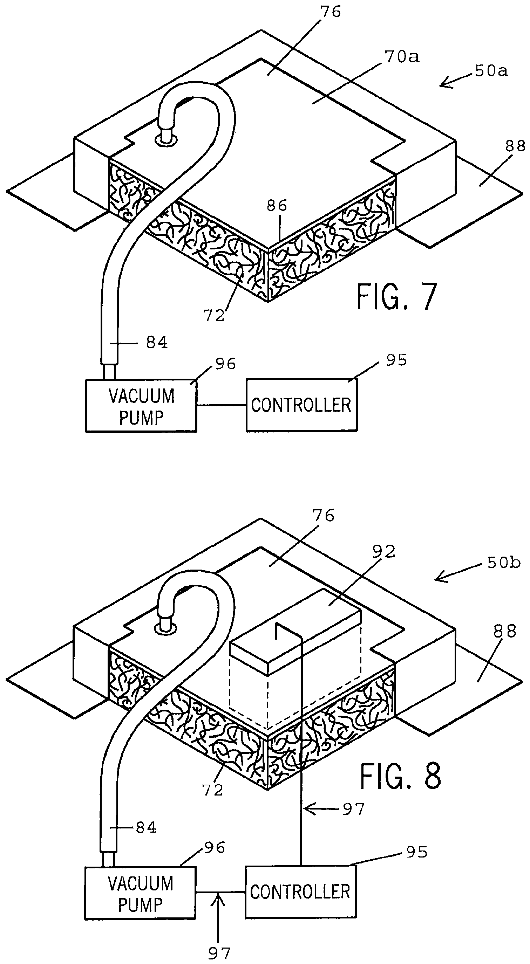

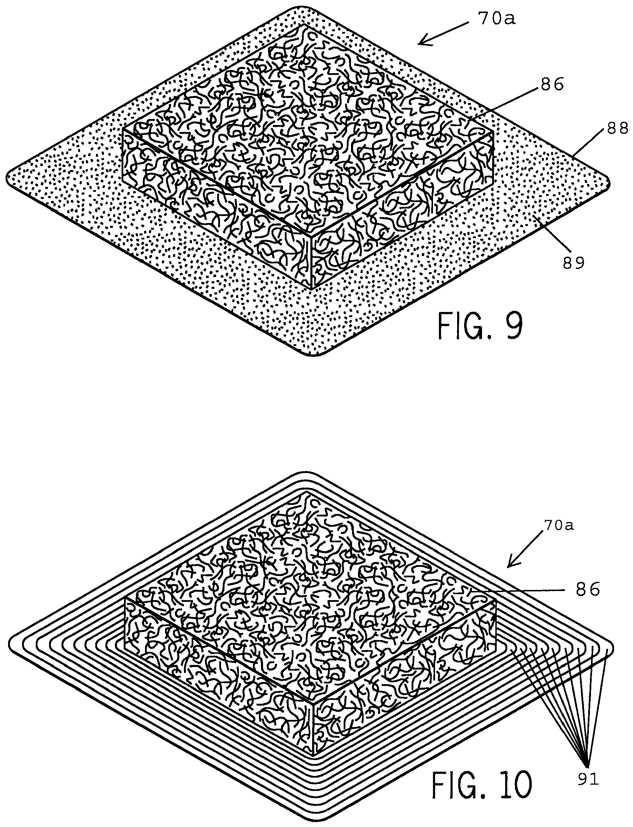

The dermal patch can be used without modifying its shape (as a pre- shaped design). Looking at Figures 7 and 9, the dermal patch 70a of the device 50a includes an outer perimeter 86. The outer perimeter 86 of the dermal patch 70a can be sealed by coating or covering with a sealing material 88 (which is shown partially broken away in Figure 7). In order to attach the dermal patch 70a to the skin and maintain vacuum, the edges in contact with the skin should be sealed. In order to achieve this, the outer perimeter 86 of the base side of the dermal patch 70a can be air sealed as the top side of the dermal patch 70a is and then coated with an adhesive 89 in Figure 9, such as following attachment to the skin the dermal patch 70a is secured to the skin in an airtight manner creating an air sealed or semi-sealed (i.e., a breathable film) space between the dermal patch 70a and the skin. Example adhesives include urethane, epoxy, urea, melamine, polyamide, polyester, polyether, saturated or unsaturated polyolefin, polyvinyl, sulfonate, acrylate or methacrylate compounds and/or combinations thereof.

Turning to Figure 10, an additional way to secure the dermal patch 70a and create an air sealed space is to apply thin layers of elastic polymer 91 with or without adhesive along the outer perimeter 86 with their direction from the dermal patch 70a base to the external skin (facing opposite from the center of the dermal patch 70a), such as when vacuum is applied the thin flexible layer or layers will be pulled in by the vacuum pressure and secure the dermal patch 70a from air leakage. The thin flexible layers can, for example, be 0.01 millimeters to 2 millimeters in width and more preferably 0.05 to 0.5 millimeters. The thin flexible layer can be fragmented or continuous along the perimeter of the dermal patch 70a forming a closed line. The thin flexible layer can be between 0.01 centimeters and 4 centimeters long as measured from their connection point to the dermal patch 70a base or more preferably between 0.5 millimeters and 10 millimeters. Such sealing configuration is shown in Figure 10.

The device can be combined with other elements to enhance blood flow. In one embodiment shown in Figure 8, the device 50b includes one or more vibrating elements 92 such as piezoelectric actuator(s), vibration motor(s), voice coil(s), electroactive polymer artificial muscle (EPAM) or other vibrating elements that will transmit vibrations to the skin or tissue. These vibrations may vary between low frequency ranging from 1 to 1000 Hz, high frequencies ranging from 1000 - 20,000 Hz or ultrasound. When a vibrating device is embedded in the dermal patch 70a, a power inlet can be attached to the dermal patch 70a and a controller 95 and power supply of the vibrating unit 92 as well as a vacuum pump 96 are connected to the dermal patch 70a through a connecting air tube 84 and electric wire 97.

The controller 95 can be programmed with various algorithms to control the vacuum pump 96. In one non-limiting example algorithm, the controller 95 executes an internally or externally program to provide a first electrical signal for a first time duration to the vacuum pump 96 for running the vacuum pump 96. After the first time duration ends, the controller 95 ceases providing the first electrical signal to the vacuum pump 96 for a second time duration such that the vacuum pump 96 does not operate. After the second time duration ends, the controller 95 resumes providing the first electrical signal to the vacuum pump 96 for a third time duration for running the vacuum pump 96. It can be appreciated that the programmable controller 95 allows for an infinite number of programs that provide for various time periods of operation or non-operation for the vacuum pump 96 and for various pump pressures. The controller 95 can also be programmed with various algorithms in stored programs to control the vibrating unit 92. Example algorithms are detailed above with reference to the controller 36.

The vibrational stimulus signal in the embodiment of Figure 8 can be a single or multiple waveforms, sinusoidal, square, pulse, triangle or combination thereof, can be unidirectional or multidirectional, and can change over time. The preferred amplitude of the vibrational stimulus signal is from about 1 µm to about 15 millimeters. The frequency of the vibrational stimulus signal is preferably in the range of about 1 Hz to about 15,000 Hz. The force of the vibrational stimulus is preferably in the range of about 0.001 Newtons to 100 Newtons.

Another embodiment of the dermal patch 70 or the dermal patch 70a includes a vibrating element, such as vibration generators 38 described earlier, and also contains a controller 95 which controls the operation of the vibrating element and a power unit which powers the vibrating element and controller all embedded within the dermal patch 70a.

In another embodiment, the dermal patch 70 or the dermal patch 70a contain a micro vacuum pump such as piezoelectric pump or motorized pump, vacuum controller, and power supply such that the dermal patch 70 or the dermal patch 70a does not need to be connected to an external vacuum pump and controller.

In another embodiment, the dermal patch 70 or the dermal patch 70a including the vibrating element also contain within the dermal patch 70 or the dermal patch 70a both the vacuum pump, controller for the vacuum pump, controller for the vibrating device and power supply for the vacuum pump, vacuum controller, vibration controller and vibrating element.

In another embodiment, the dermal patch 70 or the dermal patch 70a - with or without vibrating element(s) - also contain a heating element to transmit heat to the skin or tissue, or a cooling element to transmit cooling to the skin or tissue.

In another embodiment, the dermal patch 70 or the dermal patch 70a can be combined with an electro stimulation device embedded in the dermal patch 70 or the dermal patch 70a or connected to the dermal patch 70 or the dermal patch 70a. Electric stimulation can be a benefit in wound healing, and the use of both technologies can result in improved efficacy and better outcome. In addition to electric stimulation elements, the dermal patch 70 or the dermal patch 70a can include drug delivery element(s), massaging element(s), compression element(s), vibration element(s) and/or combinations thereof.

A kit not covered by the claims can include the dermal patch 70 or the dermal patch 70a, sealing film, the vacuum pump 96, the vacuum tubing 84, the vibration controller 95, and instructions for use. The dermal patch 70 or the dermal patch 70a can be used in combination with wound negative pressure therapy, skin system(s), skin implants, biological or bioactive wound dressings, drug delivery wound dressings, wound drainage systems and/or combinations thereof. Among other things, the dermal patch 70 or the dermal patch 70a are suitable for cosmetic use in the enhancement of skin appearance such as in the reduction of wrinkles and cellulites, in the treatment of sport injury such as muscle injuries and muscle inflammation by enhancing healing, in skin conditions by enhancing skin perfusion, in plastic and reconstructive surgery and/or in combinations thereof. The dermal patch 70 or the dermal patch 70a can include a cosmetic for cosmetic uses in the enhancement of skin appearance.

The device 50 and the device 50a have many benefits. For example, negative pressure can be used for the treatment of chronic wounds, as well as surgical incisions and other types of wounds. The technology of the present embodiments not covered by the claims targets the skin surrounding the wound and not particularly the wound itself. The technology described and not covered by the claims provides the care giver more flexibility in optimizing the size and shape of the vacuum applicator by simply cutting the dermal patch to the desired shape and attaching the vacuum dermal patch to the treated area. While vacuum applied over a wound effects blood circulation at the wound edges, the technology described and not covered by the claims allows for more substantial improvement in circulation in the skin and subdermal tissues surrounding the wound, thereby allowing for better perfusion in the wound area and better healing of the wound. The combination of a vibrating element in the dermal patch provides two complementary mechanisms for the induction of blood flow. This can result in higher efficacy due to the dual response, higher response rate to the treatment due to better chances that the patient will positively respond to one of the stimulations.

The device 50 and the device 50a can be used in combination with other devices such as compression devices, shock wave devices, heating devices, cooling devices, light emitting devices, ultrasound devices, and electric current stimulation devices.

### Examples

The following Examples have been presented in order to further illustrate the invention and are not intended to limit the invention in any way.

### Example 1

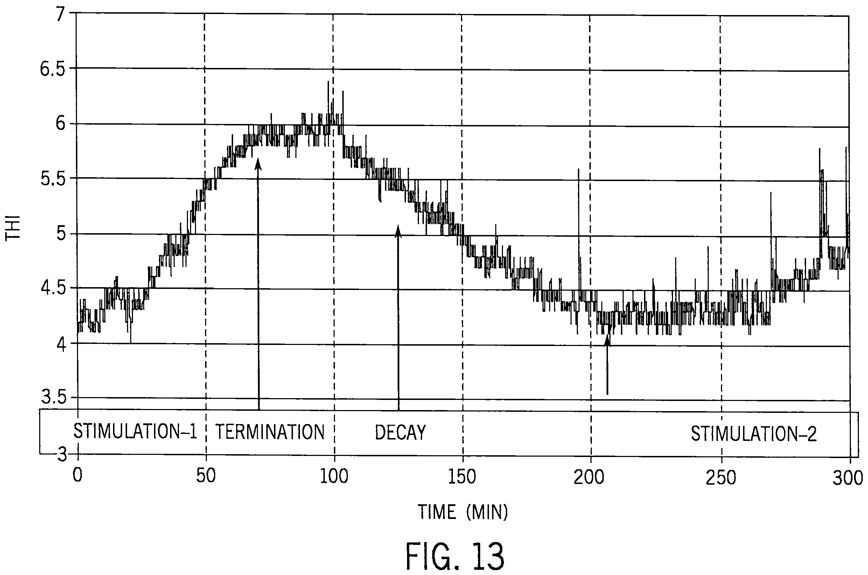

Assay method and results: A piezoelectric actuator (1 inch in diameter) was enclosed within a plastic enclosure controlled by a controller unit. The enclosure was attached to the sacrum skin of a human patient by applying adhesive tape over the actuator. After an acclimazation period of 15 to 30 minutes, vibration stimulation was started (20 Hz, 8 mils amplitude) and was continued as intermittent stimulation in 5 minute on/off cycles. THI (total hemoglobin index) was recorded versus time. After 50 minutes, the THI levels reached a plateau and the stimulation was terminated. Following a decline period and a return to baseline levels of THI, stimulation was renewed and a new cycle started at 267 minutes. The blood flow stimulation cycle can be used for the development of a continuous operation algorithm such that the device can be self regulated and increase blood flow over prolonged periods of time. For example, the plot of THI vs. time in Figure 13 teaches that an actuator with equivalent parameters of operation when attached to the skin can be activated for 50 minutes with 5 minute on/off cycles and then turned off for 200 minutes before a new cycle of stimulation is initiated. The on and off cycles can vary between subjects and between body locations. Measuring how this kinetics changes between subjects and/or body locations can be used for the development of specific algorithms for long term (hours to days) of operation. In the present study, we used the InSpectra™ StO2 Tissue Oxygenation Monitor. Similar devices or other devices used for measurement of blood flow can be used for the measurement of tissue blood flow, temperature, tissue oxygenation, or THI in the development of long term operation algorithms for use with vibration based blood flow stimulation.

### Example 2

A vibration device as in Example 1 was applied to the ankle of a human patient. The device increased blood flow in the heel and toes (prime location for diabetes and arterial foot ulcers) by over two fold as measured using moorFLPI system, a full-field video frame rate blood flow imaging system which uses a laser Doppler speckle technology.

### Example 3

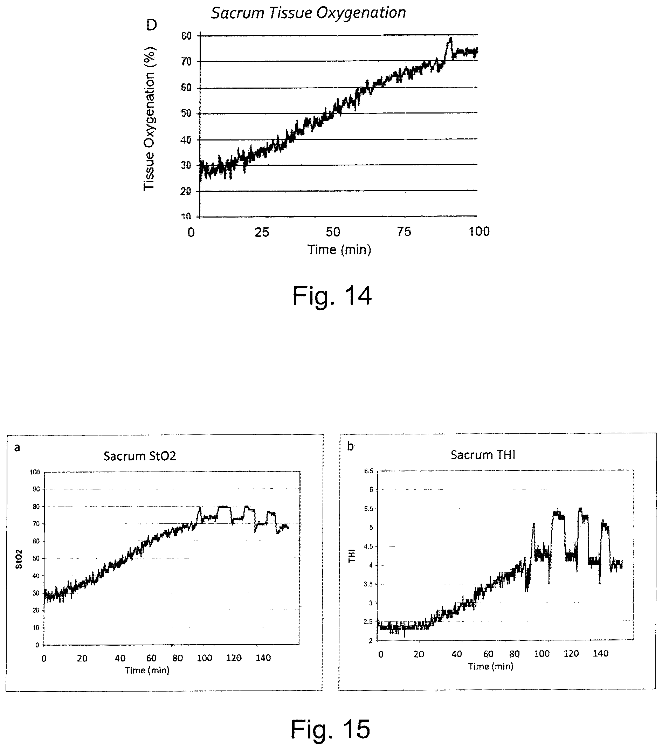

A vibration device as in Example 1 was applied to the sacrum of a human patient. The vibration device increased tissue oxygenation at the sacrum (lower back, location of 80% of pressure ulcers) by more than 2.5 fold, measured using InSpectra™ StO2 Tissue Oxygenation Monitor with the protocol similar to Example 1. See Figure 14.

### Example 4

A vacuum chamber containing a vibrating piezoelectric element was placed on the sacrum. Following a fifteen minute acclimazation period, vibration stimulation was started (20 Hz, 8 mils amplitude) and was continued as intermittent stimulation in 5 minute on/off cycles. Tissue oxygenation (StO2) (see chart a in Figure 15) and total hemoglobin (THI) (see chart b in Figure 15) increased 270% and 70% respectively. Intermittent vacuum (25 mmHg) was applied when StO2 levels were approaching a plateau resulting in an additional 10% and 20% increase in StO2 and THI respectively.

Thus, a method not covered by the claims and an apparatus are provided for manipulating local and/or regional circulation, such as inducing vasodilation or vasoconstriction, by means of vibrational stimuli and/or negative pressure. The devices of the invention can increase blood flow, increase tissue oxygenation, and increase total hemoglobin in a patient. Accordingly, it is contemplated that the devices of the invention can be beneficial in: methods for enhancing skin appearance; methods for the treatment of sport injury and other traumatic injuries by enhancing healing; methods for enhancing healing after surgery (e.g., plastic and reconstructive surgery); methods for treating peripheral artery disease or peripheral vascular disease in a subject; methods for improving wound healing in a subject; methods for increasing tissue oxygenation in a subject; methods for improving healing of a skin ulcer in a subject; methods for improving blood flow to ischemic tissue in a subject; methods for treating erectile dysfunction; methods for treating a migraine; methods for treating hair loss, methods for treating neuropathy, and methods for treating plantar fasciitis.

Although the invention has been described in considerable detail with reference to certain embodiments, one skilled in the art will appreciate that the present invention can be practiced by other than the described embodiments, which have been presented for purposes of illustration and not of limitation. Therefore, the scope of the appended claims should not be limited to the description of the embodiments contained herein.

### INDUSTRIAL APPLICABILITY

The invention provides a device for manipulating circulation in the circulatory system of the body of a subject.

## Claims

1. A device (20) for manipulating circulation in the circulatory system of the body of a subject, the device comprising:
a flexible support (22) dimensioned for covering a section of the body of the subject, the section of the body of the subject including a wound; and
a vibrating element (38) embedded in or attached to the support,
wherein the support includes a patch and an attachment layer on the patch, the attachment layer including a biocompatible adhesive suitable for attaching the patch to skin on the section of the body of the subject, and
wherein the vibrating element is positioned in or on the support such that the vibrating element can transmit vibrations to the skin when the patch is adhered to the skin on the section of the body of the subject, the vibrations of the vibrating element being at a frequency range of 1 Hz to 15,000 Hz.

2. The device of claim 1 wherein:
the support includes an opening (26) dimensioned to fully or partially surround the wound when the patch is placed over skin on the section of the body of the subject.

3. The device of claim 1 further comprising:
a power source (32); and
a controller (36) in electrical communication with the vibrating element and the power source, the controller being configured to execute a program stored in the controller to provide an electrical signal to the vibrating element, the electrical signal controlling timing, frequency, amplitude, waveform, force and/or other parameters of operation of the vibrating element, the electrical signal being (i) a single or multiple waveform, (ii) unidirectional or multidirectional, and (iii) sinusoidal, square, pulse, triangle or a combination thereof.

4. The device of claim 3 wherein:
the controller (36) is further configured to execute the program to:
(v) cease providing the electrical signal to the vibrating element or decrease intensity of the electrical signal provided to the vibrating element or increase intensity of the electrical signal provided to the vibrating element for a first time duration, and
(vi) resume providing the electrical signal to the vibrating element or increase intensity of the electrical signal provided to the vibrating element or decrease intensity of the electrical signal provided to the vibrating element for a second time duration.

5. The device of claim 1 further comprising:
a second vibrating element (38) embedded in or attached to the support;
a power source (32); and
a controller (36) in electrical communication with the first vibrating element and the second vibrating element and the power source, the controller being configured to execute a program to:
(i) provide a first electrical signal for a first time duration to the vibrating element, the first electrical signal controlling the frequency and amplitude and force of vibrations of the vibrating element,
(ii) cease providing the first electrical signal to the vibrating element for a second time duration, and
(iii) provide a second electrical signal to the second vibrating element for a third time duration, the second electrical signal controlling the frequency and amplitude and force of vibrations of the second vibrating element.

6. The device of claim 1 further comprising:
a power source (32); and
a controller (36) in electrical communication with the vibrating element and the power source, the controller being configured to execute a program to:
(i) provide a first electrical signal to the vibrating element, the first electrical signal controlling the frequency and amplitude and force of vibrations of the vibrating element, and
(iii) vary the first electrical signal to the vibrating element such that at least one of the frequency or amplitude or force of vibrations of the vibrating element is varied.

7. The device of claim 1 further comprising:
a perfusion sensor for covering a second section of the body of the subject, the second section being adjacent to or within the first section of the body of the subject;
a power source (32); and
a controller (36) in electrical communication with the vibrating element and the power source and the perfusion sensor, the controller being configured to execute a program stored in the controller to:
(i) provide a first electrical signal for a first time duration to the vibrating element, the first electrical signal controlling the frequency and amplitude and force of vibrations of the vibrating element, and
(ii) vary the first electrical signal provided to the vibrating element based on a second electrical signal received from the perfusion sensor.

8. The device of claim 7 wherein:
the perfusion sensor is selected from piezo-film sensors, temperature sensors, photoplethysmographic sensors, strain-gauge plethysmography sensors, laser Doppler sensors, laser speckle sensors, infra-red imaging, infra-red spectroscopy, and ultrasound sensors.

9. The device of claim 1 further comprising:
a vibration sensor for covering a second section of the body of the subject, the second section being adjacent to or within the first section of the body of the subject;
a power source (32); and
a controller (36) in electrical communication with the vibrating element and the power source and the vibration sensor, the controller being configured to execute a program stored in the controller to:
(i) provide a first electrical signal for a first time duration to the vibrating element, the first electrical signal controlling the frequency and amplitude and force of vibrations of the vibrating element, and
(ii) vary the first electrical signal provided to the vibrating element based on a second electrical signal received from the vibration sensor.

10. The device of any of claims 1 to 9 wherein:
the support comprises a patch formed from a material selected from silicone, synthetic foam, polyethylene, polyurethane, polyvinyl chloride, plastic, thermoplastic polyurethane, polypropylene, fabric, hydrogel, collagen, alginate, gelatin, or combinations thereof.

11. The device of any of claims 1 to 10 wherein:
each vibrating element is selected from linear electromagnetic actuators, asymmetric mass motors, voice coils, electro-active polymers, piezoelectric actuators, fluid-containing resonating elements, gas-containing resonating elements, and combinations thereof.

12. The device of any of claims 1 to 11 wherein:
the vibrations have a force ranging from 0.001 Newtons to 100 Newtons.

13. The device of any of claims 1 to 12 wherein:
the wound is selected from the group consisting of diabetic ulcers, ischemic ulcers, venous ulcers, arterial ulcers, ischemic wounds, pressure wounds, injuries, surgeries, surgical incisions, surgical wounds, burns, inflammation, muscle injuries, muscle inflammation, musculo-skeletal injuries, and internal injuries, and
the device further includes a hydrogel matrix selected from polylactic acid, polyglycolic acid, other polyhydroxy acids, copolymers of two or more polyhydroxy acids, polyorthoesters, polyanhydrides, gelatin, collagen, cellulose, derivatized cellulose, chitosan, alginate, thiol-modified hyaluronan, emulsion, and combinations or copolymers thereof for covering the wound.

14. The device of any of claims 1 to 13 further comprising:
at least one of: devices for applying negative pressure below or adjacent to the support, hyperbaric oxygen devices, compression devices, shock wave devices, heating devices, cooling devices, light emitting devices, ultrasound devices, electro-magnetic stimulation devices, magnetic stimulation devices and electric current stimulation devices.

15. The device of any of claims 1 to 11 wherein:
the vibrations of the vibrating element have an amplitude ranging from 0.001 to 2.5 millimeters.

## Patentansprüche

1. Vorrichtung (20) zur Beeinflussung des Kreislaufs in dem Kreislaufsystem des Körpers eines Patienten, wobei die Vorrichtung Folgendes umfasst:
eine flexible Auflage (22), welche so bemessen ist, dass sie einen Abschnitt des Körpers des Patienten bedeckt, wobei der Abschnitt des Körpers des Patienten eine Wunde umfasst; und
ein Schwingungselement (38), welches in die Auflage eingebettet oder an dieser befestigt ist,
wobei die Auflage ein Pflaster und eine Haftschicht auf dem Pflaster umfasst, wobei die Haftschicht einen biokompatiblen Klebstoff umfasst, der geeignet ist, das Pflaster an der Haut auf dem Abschnitt des Körpers des Patienten zu befestigen, und
wobei das Schwingungselement derart in oder an der Auflage angeordnet ist, dass bei an der Haut auf dem Abschnitt des Körpers des Patienten haftendem Pflaster durch das Schwingungselement Schwingungen auf die Haut übertragbar sind, wobei die Schwingungen des Schwingungselements in einem Frequenzbereich von 1 Hz bis 15 000 Hz liegen.

2. Vorrichtung nach Anspruch 1, wobei:
die Auflage eine Öffnung (26) umfasst, die derart bemessen ist, dass sie die Wunde vollständig oder teilweise umgibt, wenn das Pflaster auf der Haut des Abschnitts des Körpers des Patienten aufgebracht ist.

3. Vorrichtung nach Anspruch 1, des Weiteren umfassend:
eine Stromquelle (32); und
eine Steuerung (36), die mit dem Schwingungselement und der Stromquelle in elektrischer Verbindung steht, wobei die Steuerung dazu ausgebildet ist, zur Bereitstellung eines elektrischen Signals für das Schwingungselement ein in der Steuerung gespeichertes Programm auszuführen, wobei durch das elektrische Signal das Timing, die Frequenz, die Amplitude, die Wellenform, die Kraft und/oder weitere Parameter des Betriebs des Schwingungselements steuerbar sind, wobei das elektrische Signal (i) eine Einzel- oder Mehrfachwellenform aufweist, (ii) unidirektional oder multidirektional und (iii) sinusförmig, rechteckig, gepulst, dreieckig oder eine Kombination derselben ist.

4. Vorrichtung nach Anspruch 3, wobei:
die Steuerung (36) des Weiteren zur Ausführung des Programms ausgebildet ist, um:
(v) für einen ersten Zeitraum die Bereitstellung des elektrischen Signals für das Schwingungselement einzustellen oder die Intensität des dem Schwingungselement bereitgestellten Signals zu verringern oder die Intensität des dem Schwingungselement bereitgestellten Signals zu erhöhen, und
(vi) für einen zweiten Zeitraum die Bereitstellung des elektrischen Signals für das Schwingungselement wieder aufzunehmen oder die Intensität des dem Schwingungselement bereitgestellten Signals zu erhöhen oder die Intensität des dem Schwingungselement bereitgestellten Signals zu verringern.

5. Vorrichtung nach Anspruch 1, des Weiteren umfassend:
ein zweites Schwingungselement (38), welches in die Auflage eingebettet oder an dieser befestigt ist;
eine Stromquelle (32); und
eine Steuerung (36), die mit dem ersten Schwingungselement und dem zweiten Schwingungselement und der Stromquelle in elektrischer Verbindung steht, wobei die Steuerung zur Ausführung eines Programms ausgebildet ist, um:
(i) dem Schwingungselement für einen ersten Zeitraum ein erstes elektrisches Signal bereitzustellen, wobei durch das erste elektrische Signal die Frequenz und die Amplitude und die Kraft der Schwingungen des Schwingungselements steuerbar sind,
(ii) die Bereitstellung des ersten elektrischen Signals für das Schwingungselement für einen zweiten Zeitraum einzustellen, und
(iii) dem Schwingungselement für einen dritten Zeitraum ein zweites elektrisches Signal bereitzustellen, wobei durch das zweite elektrische Signal die Frequenz und die Amplitude und die Kraft der Schwingungen des zweiten Schwingungselements steuerbar sind.

6. Vorrichtung nach Anspruch 1, des Weiteren umfassend:
eine Stromquelle (32); und
eine Steuerung (36), die mit dem Schwingungselement und der Stromquelle in elektrischer Verbindung steht, wobei die Steuerung zur Ausführung eines Programms ausgebildet ist, um:
(i) dem Schwingungselement ein erstes elektrisches Signal bereitzustellen, wobei durch das erste elektrische Signal die Frequenz und die Amplitude und die Kraft der Schwingungen des Schwingungselements steuerbar sind, und
(iii) das elektrische Signal an das Schwingungselement zu variieren, so dass die Frequenz und/oder die Amplitude und/oder die Kraft der Schwingungen des Schwingungselements variierbar sind.

7. Vorrichtung nach Anspruch 1, des Weiteren umfassend:
einen Perfusionssensor zum Bedecken eines zweiten Abschnitts des Körpers des Patienten, wobei der zweite Abschnitt dem ersten Abschnitt des Körpers des Patienten benachbart oder in diesem angeordnet ist;
eine Stromquelle (32); und
eine Steuerung (36), die mit dem Schwingungselement und der Stromquelle und dem Perfusionssensor in elektrischer Verbindung steht, wobei die Steuerung zur Ausführung eines in der Steuerung gespeicherten Programms ausgebildet ist, um:
(i) dem Schwingungselement für einen ersten Zeitraum ein erstes elektrisches Signal bereitzustellen, wobei durch das erste elektrische Signal die Frequenz und die Amplitude und die Kraft der Schwingungen des Schwingungselements steuerbar sind, und
(ii) das dem Schwingungselement bereitgestellte erste Signal auf Grundlage eines von dem Perfusionssensor empfangenen zweiten elektrischen Signals zu variieren.

8. Vorrichtung nach Anspruch 7, wobei:
der Perfusionssensor aus Piezo-Foliensensoren, Temperatursensoren, Fotoplethysmografiesensoren, Strain-Gauge-Plethysmografiesensoren, Laser-Doppler-Sensoren, Laser-Speckle-Sensoren, Infrarot-Bildgebung, Infrarot-Spektroskopie und Ultraschallsensoren ausgewählt ist.

9. Vorrichtung nach Anspruch 1, des Weiteren umfassend:
einen Schwingungssensor zum Bedecken eines zweiten Abschnitts des Körpers des Patienten, wobei der zweite Abschnitt dem ersten Abschnitt des Körpers des Patienten benachbart oder in diesem angeordnet ist;
eine Stromquelle (32); und
eine Steuerung (36), die mit dem Schwingungselement und der Stromquelle und dem Schwingungssensor in elektrischer Verbindung steht, wobei die Steuerung zur Ausführung eines in der Steuerung gespeicherten Programms ausgebildet ist, um:
(i) dem Schwingungselement für einen ersten Zeitraum ein erstes elektrisches Signal bereitzustellen, wobei durch das erste elektrische Signal die Frequenz und die Amplitude und die Kraft der Schwingungen des Schwingungselements steuerbar sind, und
(ii) das dem Schwingungselement bereitgestellte erste Signal auf Grundlage eines von dem Schwingungssensor empfangenen zweiten elektrischen Signals zu variieren.

10. Vorrichtung nach einem der Ansprüche 1 bis 9, wobei:
die Auflage ein Pflaster umfasst, das aus einem aus Silikon, Kunstschaum, Polyethylen, Polyurethan, Polyvinylchlorid, Plastik, thermoplastischem Polyurethan, Polypropylen, Stoff, Hydrogel, Kollagen, Alginat, Gelatine oder einer Kombination derselben ausgewählten Material gebildet ist.

11. Vorrichtung nach einem der Ansprüche 1 bis 10, wobei:
die Schwingungselemente jeweils aus linearen elektromagnetischen Aktoren, asymmetrischen Massemotoren, Schwingspulen, elektroaktiven Polymeren, piezoelektrischen Aktoren, fluidhaltigen Resonanzelementen, gashaltigen Resonanzelementen und Kombinationen derselben ausgewählt sind.

12. Vorrichtung nach einem der Ansprüche 1 bis 11, wobei:
die Schwingungen eine Kraft von 0,001 Newton bis 100 Newton aufweisen.

13. Vorrichtung nach einem der Ansprüche 1 bis 12, wobei:
die Wunde aus der Gruppe bestehend aus diabetischen Ulcera, ischämischen Ulcera, venösen Ulcera, arteriellen Ulcera, ischämischen Wunden, Druckwunden, Verletzungen, Operationen, chirurgischen Inzisionen, chirurgischen Wunden, Verbrennungen, Entzündungen, Muskelverletzungen, Muskelentzündungen, Muskel-Skelett-Verletzungen und inneren Verletzungen ausgewählt ist, und
die Vorrichtung des Weiteren eine aus Polymilchsäure, Polyglycolsäure, anderen Polyhydroxysäuren, Copolymeren aus zwei oder mehr Polyhydroxysäuren, Polyorthoestern, Polyanhydriden, Gelatine, Kollagen, Cellulose, derivatisierter Cellulose, Chitosan, Alginat, thiolmodifiziertem Hyaluronan, Emulsion und Kombinationen oder Copolymeren derselben ausgewählte Hydrogel-Matrix zum Abdecken der Wunde umfasst.

14. Vorrichtung nach einem der Ansprüche 1 bis 13, des Weiteren umfassend:
mindestens eine der Folgenden: Vorrichtungen zum Anlegen eines Unterdrucks unter oder benachbart der Auflage, hyperbare Sauerstoffvorrichtungen, Kompressionsvorrichtungen, Stoßwellenvorrichtungen, Heizvorrichtungen, Kühlvorrichtungen, Licht emittierende Vorrichtungen, Ultraschallvorrichtungen, elektromagnetische Stimulationsvorrichtungen, magnetische Stimulationsvorrichtungen und Stromstimulationsvorrichtungen.

15. Vorrichtung nach einem der Ansprüche 1 bis 11, wobei:
die Schwingungen des Schwingungselements eine Amplitude von 0,001 bis 2,5 Millimetern aufweisen.

## Revendications

1. Dispositif (20) pour la manipulation de la circulation dans le système circulatoire du corps d'un patient, le dispositif comprenant :
un support flexible (22) dimensionné pour couvrir une partie du corps du patient, la partie du corps du patient contenant une plaie ; et
un élément vibrant (38) incorporé dans le ou fixé au support,
dans lequel le support comprend un patch et une couche de fixation sur le patch, la couche de fixation comprenant un adhésif biocompatible approprié à fixer le patch à la peau sur la partie du corps du patient, et
dans lequel l'élément vibrant est disposée dans ou sur le support de telle manière que l'élément vibrant peut transmettre des vibrations à la peau quand le patch adhère à la peau sur la partie du corps du patient, les vibrations de l'élément vibrant étant dans une gamme de fréquence de 1 Hz à 15 000 Hz.

2. Dispositif selon la revendication 1, dans lequel :
le support comprend une ouverture (26) dimensionnée pour entourer entièrement ou partiellement la plaie quand le patch est placé sur la peau sur la partie du corps du patient.

3. Dispositif selon la revendication 1, comprenant en outre :
une source d'énergie (32) ; et
un contrôleur (36) en communication électrique avec l'élément vibrant et la source d'énergie, le contrôleur étant configuré pour exécuter un programme enregistré dans le contrôleur afin de fournir un signal électrique à l'élément vibrant, le signal électrique contrôlant le timing, la fréquence, l'amplitude, la forme d'onde, la force et/ou d'autres paramètres du fonctionnement de l'élément vibrant, le signal électrique étant (i) une forme d'onde seule ou multiple, (ii) unidirectionnel ou multidi-rectionnel, et (iii) sinusoïdal, carré, pulsé, triangulaire ou une combinaison de ce qui précède.

4. Dispositif selon la revendication 3, dans lequel :
le contrôleur (36) est configuré en outre pour exécuter le programme afin de :
(v) cesser de fournir le signal électrique à l'élément vibrant ou réduire l'intensité du signal électrique fourni à l'élément vibrant ou augmenter l'intensité du signal électrique fourni à l'élément vibrant pour une première durée de temps, et
(vi) continuer de fournir le signal électrique à l'élément vibrant ou augmenter l'intensité du signal électrique fourni à l'élément vibrant ou réduire l'intensité du signal électrique fourni à l'élément vibrant pour une deuxième durée de temps.

5. Dispositif selon la revendication 1, comprenant en outre :
un deuxième élément vibrant (38) incorporé dans le ou fixé au support ;
une source d'énergie (32) ; et
un contrôleur (36) en communication électrique avec le premier élément vibrant et le deuxième élément vibrant et la source d'énergie, le contrôleur étant configuré pour exécuter un programme afin de :
(i) fournir un premier signal électrique pour une première durée de temps à l'élément vibrant, le premier signal électrique contrôlant la fréquence et l'amplitude et la force des vibrations de l'élément vibrant,
(ii) cesser de fournir le premier signal électrique à l'élément vibrant pour une deuxième durée de temps, et
(iii) fournir un deuxième signal électrique au deuxième élément vibrant pour une troisième durée de temps, le deuxième signal électrique contrôlant la fréquence et l'amplitude et la force des vibrations du deuxième élément vibrant.

6. Dispositif selon la revendication 1, comprenant en outre :
une source d'énergie (32) ; et
un contrôleur (36) en communication électrique avec l'élément vibrant et la source d'énergie, le contrôleur étant configuré pour exécuter un programme afin de :
(i) fournir un premier signal électrique à l'élément vibrant, le premier signal électrique contrôlant la fréquence et l'amplitude et la force des vibrations de l'élément vibrant, et
(iii) varier le premier signal électrique à l'élément électrique de telle manière qu'au moins une de la fréquence ou l'amplitude ou la force des vibrations de l'élément vibrant est variée.

7. Dispositif selon la revendication 1, comprenant en outre :
un capteur de perfusion pour couvrir une deuxième partie du corps du patient, la deuxième partie étant adjacente à ou dans la première partie du corps du patient ; une source d'énergie (32) ; et
un contrôleur (36) en communication électrique avec l'élément vibrant et la source d'énergie et le capteur de perfusion, le contrôleur étant configuré pour exécuter un programme enregistré dans le contrôleur afin de :
(i) fournir un premier signal électrique pour une première durée de temps à l'élément vibrant, le premier signal électrique contrôlant la fréquence et l'amplitude et la force des vibrations de l'élément vibrant, et
(ii) varier le premier signal électrique fourni à l'élément vibrant sur la base d'un deuxième signal électrique reçu du capteur de perfusion.

8. Dispositif selon la revendication 7, dans lequel :
le capteur de perfusion est sélectionné parmi les capteurs à film piézoélectrique, les capteurs de température, les capteurs photopléthysmographiques, les capteurs de photopléthysmographie à jauge de contrainte, les capteurs laser-doppler, les capteurs de tavelures de laser, l'imagerie infrarouge, la spectroscopie infrarouge et les capteurs à ultrasons.

9. Dispositif selon la revendication 1, comprenant en outre :
un capteur de vibrations pour couvrir une deuxième partie du corps du patient, la deuxième partie étant adjacente à ou dans la première partie du corps du patient ;
une source d'énergie (32) ; et
un contrôleur (36) en communication électrique avec l'élément vibrant et la source d'énergie et le capteur de vibrations, le contrôleur étant configuré pour exécuter un programme enregistré dans le contrôleur afin de :
(i) fournir un premier signal électrique pour une première durée de temps à l'élément vibrant, le premier signal électrique contrôlant la fréquence et l'amplitude et la force des vibrations de l'élément vibrant, et
(ii) varier le premier signal électrique fourni à l'élément vibrant sur la base d'un deuxième signal électrique reçu du capteur de vibrations.

10. Dispositif selon l'une quelconque des revendications 1 à 9, dans lequel :
le support comprend un patch formé à partir d'un matériau sélectionné parmi la silicone, la mousse synthétique, le polyéthylène, le polyuréthane, le chlorure de poly-vinyle, le plastique, le polyuréthane thermoplastique, le polypropylène, le tissu, l'hydrogel, le collagène, l'alginate, la gélatine ou des combinaisons de ce qui précède.

11. Dispositif selon l'une quelconque des revendications 1 à 10, dans lequel :
chaque élément vibrant est sélectionné parmi les actionneurs électromagnétiques linéaires, les moteurs à masse asymétrique, les bobines acoustiques, les polymères électro-actifs, les actionneurs piézoélectriques, les éléments résonants contenant du fluide, les éléments résonant contenant du gaz et des combinaisons de ce qui précède.

12. Dispositif selon l'une quelconque des revendications 1 à 11, dans lequel :
les vibrations ont une force allant de 0,001 Newtons à 100 Newtons.

13. Dispositif selon l'une quelconque des revendications 1 à 12, dans lequel :
la plaie est sélectionnée dans le groupe consistant en les ulcères diabétiques, les ulcères ischémiques, les ulcères veineux, les ulcères artériels, les plaies ischémiques, les plaies de pression, les blessures, les opérations, les incisions chirurgicales, les plaies chirurgicales, les brûlures, l'inflammation, les blessures musculaires, l'inflammation musculaire, les blessures musculosquelettiques et les lésions internes, et
le dispositif comprend en outre une matrice d'hydrogel sélectionnée parmi l'acide polylactique, l'acide polyglycolique, autres acides polyhydroxiques, les copolymères de deux ou plusieurs acides polyhydroxiques, les polyorthoesters, les polyanhydrides, la gélatine, le collagène, la cellulose, la cellulose dérivatisée, le chitosan, l'alginate, l'acide hyaluronique modifié par le thiol, une émulsion et des combinaisons ou copolymères de ce qui précède pour couvrir la plaie.

14. Dispositif selon l'une quelconque des revendications 1 à 13, comprenant en outre :
au moins un de : des dispositifs pour appliquer une pression négative au-dessous du ou adjacent au support, des dispositifs à oxygène hyperbare, des dispositifs de compression, des dispositifs d'ondes de choc, des dispositifs chauffants, des dispositifs refroidissants, des dispositifs émetteurs de lumière, des dispositifs à ultrason, des dispositifs de stimulation électromagnétique, des dispositifs de stimulation magnétique et des dispositifs de stimulation à courant électrique.

15. Dispositif selon l'une quelconque des revendications 1 à 11, dans lequel :
les vibrations de l'élément vibrant ont une amplitude allant de 0,001 à 2,5 millimètres.
